# FASCICULE DE BREVET EUROPEEN

(11) **EP 3 187 166 B1**
(45) Date de publication et mention de la délivrance du brevet: **17.03.2021**
(21) Numéro de dépôt: 16206904.1
(22) Date de dépôt: 26.12.2016
(51) Int. Cl.: A61K 8/37, A61Q 5/00, A61Q 19/00, A61K 8/85, C08G 63/553

(54) **POLYESTER EPAISSISSANT ET/OU RHEOFLUIDIFIANT**
VERDICKENDES UND/ODER DILATANTES POLYESTER
THICKENING AND/OR SHEAR-THINNING POLYESTER

(30) Priorité: 31.12.2015 FR 1563498
(43) Date de publication de la demande: 05.07.2017
(73) Titulaire: Oleon N.V., 9940 Ertvelde (BE)
(72) Inventeur: PEETERS, Hilde, 3140 KEERBERGEN (BE); CANIVENC, Edith, 60200 COMPIEGNE (FR)
(74) Mandataire: Santarelli

(56) Documents cités:
- EP-A1- 2 460 864
- US-A- 3 674 727
- US-A- 4 853 430
- US-A1- 2010 047 194

## Description

La présente demande se rapporte à un polyester, à son procédé de préparation et à ses utilisations comme épaississant et/ou rhéofluidifiant de matières grasses, en particulier comme épaississant rhéofluidifiant thixotrope dans le domaine de la cosmétique.

Une matière grasse peut avoir de nombreuses propriétés, et de ce fait peut être utilisée dans de nombreux domaines, tels que par exemple en cosmétique, où elle peut être utilisée comme émollient ou tensioactif, ou par exemple en peinture, où elle peut être utilisée comme diluant ou également comme tensioactif.

Plus particulièrement, dans la présente demande, par matière grasse, on entend une matière hydrophobe possédant une chaîne hydrocarbonée saturée ou insaturée comportant au moins quatre atomes de carbone et au moins une fonction alcool, acide ou ester. Généralement, ces matières grasses sont liquides à 25°C et à pression atmosphérique, et ont besoin d'être épaissies pour faciliter leur utilisation.

Pour épaissir, on utilise généralement un épaississant, c'est à dire un composé qui augmente la viscosité d'un produit dans lequel il est incorporé. Dans la présente demande, on utilisera indifféremment les termes épaississant et viscosifiant.

De manière conventionnelle, on utilise la silice ou des argiles pour épaissir les matières grasses.

Les problèmes associés à l'utilisation de tels épaississants sont la perte de brillant du produit final, ainsi que la poussière engendrée.

La demande de brevet US 2010/047194 décrit des polyesters utiles comme structurants de phases huileuses. Le terme structurant y est défini comme ayant la propriété d'augmenter la viscosité d'une huile, comme cela est démontré dans les exemples utilisant entre autres comme huiles, des alcools gras et des esters gras. Ces polyesters sont obtenus par réaction entre un polyol, un dimère d'acide et un acide monocarboxylique à longue chaîne.

Cependant, il a pu être constaté que le pouvoir épaississant de tels polyesters sur des émulsions est insuffisant.

Le travail des inventeurs a permis de mettre en évidence que certains polyesters particuliers permettaient non seulement d'épaissir des matières grasses, mais permettaient de manière tout à fait surprenante d'augmenter la viscosité d'émulsions.

La présente invention vise un polyester susceptible d'être obtenu par un procédé comportant une étape de mise en réaction de plusieurs réactifs, les réactifs comportant :
- au moins un polyol,
- au moins un acide dicarboxylique autre qu'un dimère d'acide, et
- au moins un trimère d'acide en mélange ou non avec au moins un dimère d'acide, étant entendu que si un dimère d'acide est présent, le mélange de trimère d'acide et de dimère d'acide contient au moins 50% en poids de trimère d'acide, le pourcentage en poids étant donné sur le poids total dudit mélange,
la teneur en trimère d'acide étant supérieure ou égale à 5% en poids, le pourcentage en poids étant donné sur le poids total des réactifs mis en réaction.

Le polyester selon l'invention est solide. Il peut avantageusement être mis sous forme particulaire pour en faciliter sa manipulation.

Par polyol, on entend une chaîne hydrocarbonée, linéaire, ramifiée ou cyclique, substituée par au moins deux fonctions hydroxyles, et pouvant comporter éventuellement un ou plusieurs atome d'oxygène en tant qu'hétéroatome formant des éthers (C-O-C), et pouvant éventuellement être substituée par un autre groupe tel un groupe carboxylique (COOH).

A titre d'exemple, on peut citer comme polyols, le 1,2-éthanediol ou éthylène glycol, le 1,6-hexanediol, le 1,2-propanediol ou propylène glycol, le 1,3-propanediol, le 1,4-butanediol, le 2-butyl-2-éthyl-1,3-propanediol, le 2-méthyl-1,3-propanediol, le néopentylglycol, le pentaérythritol, le di-pentaérythritol, les dimères de diol, les polydimères de diol, le polypropylène glycol, l'acide diméthylolpropionique, et l'isosorbide.

Par diol, on entend un polyol substitué par uniquement deux fonctions hydroxyles, et pouvant comporter éventuellement un ou plusieurs atome d'oxygène en tant qu'hétéroatome formant des éthers (C-O-C), et pouvant éventuellement être substitué par une autre fonction telle qu'une fonction carboxylique (CO₂H).

Par dimère de diol, on entend un composé ou un mélange de composé susceptible d'être obtenu par dimérisation d'au moins un acide carboxylique insaturé, en particulier choisi parmi les acides gras insaturés comportant de 16 à 24 atomes de carbone, suivi d'une hydrogénation réduisant les fonctions acides en fonctions alcools. Généralement, un dimère de diol est constitué d'un mélange d'isomères pouvant chacun avoir une structure acyclique, monocyclique, bicyclique et/ou aromatique.

De préférence, le dimère de diol comporte de 32 à 48 atomes de carbone, de préférence de 36 à 44 atomes de carbone.

De manière avantageuse, le dimère de diol résulte de la dimérisation d'un ou plusieurs acides monocarboxyliques comportant de 16 à 24 atomes de carbone et de 1 à 3 insaturations.

De manière particulièrement préférée, le dimère de diol résulte de la dimérisation de l'acide oléique.

Par polydimère de diol, on entend un composé ou un mélange de composé issu de la polymérisation d'un dimère de diol, tel que défini ci-dessus. Il est constitué d'au moins deux unités de dimère de diol reliées par une fonction éther.

De préférence, le polyol mis en oeuvre dans le procédé d'obtention du polyester, comporte uniquement des groupes hydroxyles primaires (de type -CH₂OH).

De préférence, le polyol est un diol, en particulier un diol dont les groupes hydroxyles sont primaires.

De préférence, les réactifs comportent au moins deux polyols et, de manière particulièrement préférée, au moins un polyol est un diol, en particulier, un diol dont les groupes hydroxyles sont primaires.

Avantageusement, le ou les polyol(s) est/sont d'origine animale, végétale ou microbienne.

De préférence, le polyol ou les polyols est/sont choisi(s) parmi le groupe constitué par le 1,6-hexanediol, le 1,3-propanediol, 2-butyl-2-éthyl-1,3-propanediol, 2-méthyl-1,3-propanediol, le néopentylglycol, l'acide diméthylolpropionique, le pentaérythritol, le di-pentaérythritol, les dimères de diol, les polydimères de diol, notamment les dimères de diol et les polydimères de diol décrits ci-dessus, et leurs mélanges.

Plus préférentiellement, le polyol est choisi dans le groupe constitué par le 1,6-hexanediol, les dimères de diol, notamment les dimères de diol décrits ci-dessus, en particulier ceux résultant de la dimérisation de l'acide oléique, et leurs mélanges.

Par acide dicarboxylique, on entend une chaîne hydrocarbonée comportant deux groupes carboxyliques, à l'exception des dimères d'acides.

De préférence, la chaîne hydrocarbonée de l'acide dicarboxylique est linéaire. Dans ce cas, les groupes carboxyliques sont avantageusement situés aux deux bouts de ladite chaîne.

De préférence, l'acide dicarboxylique est saturé.

A titre d'exemple d'acide dicarboxylique, on peut citer l'acide malonique (ou acide propanedioique), l'acide succinique, l'acide glutarique, l'acide adipique, l'acide pimélique, l'acide subérique, l'acide azélaïque, l'acide sébacique, l'acide brassilique, l'acide undecanedioïque et l'acide dodecanedioïque.

Avantageusement, l'acide dicarboxylique est d'origine animale, végétale ou microbienne.

De préférence, l'acide dicarboxylique est choisi parmi le groupe constitué par l'acide adipique, l'acide subérique, l'acide azélaïque, l'acide sébacique, l'acide dodecanedioïque et leurs mélanges.

Par dimère d'acide, on entend un acide dicarboxylique ou un mélange d'acides dicarboxyliques, obtenu par dimérisation d'acide monocarboxylique insaturé, en particulier d'acide gras insaturé comportant de 16 à 24 atomes de carbone. Un dimère d'acide est généralement constitué d'un mélange d'isomères pouvant chacun avoir une structure acyclique, monocyclique, bicyclique et/ou aromatique.

Les acides monocarboxyliques formant le dimère d'acide peuvent être identiques ou différents. Si les acides monocarboxyliques sont différents, alors le dimère d'acide comprend un mélange de dimères résultant de la dimérisation d'un même acide monocarboxylique et de la dimérisation de deux acides carboxyliques différents.

De préférence, le dimère d'acide comporte de 32 à 48 atomes de carbone, plus préférentiellement, de 36 à 44 atomes de carbone.

Par trimère d'acide, on entend un acide tricarboxylique ou un mélange d'acides tricarboxyliques obtenu par trimérisation d'acide monocarboxylique insaturé, en particulier d'acide gras insaturé comportant de 16 à 24 atomes de carbone. Un trimère d'acide est généralement un sous-produit de la réaction de dimérisation d'acide. Il est récupéré dans le distillat, après distillation du dimère d'acide.

Un trimère d'acide est généralement constitué d'un mélange d'isomères pouvant chacun avoir une structure acyclique, monocyclique, bicyclique et/ou aromatique.

Les acides monocarboxyliques formant le trimère d'acide peuvent être chacun identiques ou différents. Si les acides monocarboxyliques sont différents, alors le trimère d'acide comprend un mélange de trimère résultant de la trimérisation d'un même acide monocarboxylique et de la trimérisation d'un acide monocarboxylique avec au moins un acide monocarboxylique différent.

De préférence, le trimère d'acide comporte de 48 à 72 atomes de carbone, plus préférentiellement, de 54 à 66 atomes de carbone.

Lorsque le polyester conforme à l'invention résulte de la mise en oeuvre d'un mélange de trimère d'acide et de dimère d'acide, alors la teneur en trimère d'acide dans ledit mélange varie de 50% à 100% en poids, le pourcentage étant donné sur le poids total dudit mélange. Cette teneur peut être augmentée en effectuant une distillation du dimère d'acide.

De préférence, le mélange de trimère d'acide et de dimère d'acide contient au moins 60% en poids de trimère d'acide, plus préférentiellement au moins 70% en poids, plus préférentiellement encore au moins 80% en poids, le pourcentage étant donné sur le poids total du mélange.

Avantageusement, le mélange de trimère d'acide et de dimère d'acide est hydrogéné. Le polyester obtenu à partir d'un mélange de trimère d'acide et de dimère d'acide hydrogéné, est moins coloré.

Avantageusement, le trimère d'acide et le dimère d'acide sont d'origine animale, végétale ou microbienne.

Le trimère d'acide et le dimère d'acide sont préparés à partir d'au moins un acide gras insaturé ou d'un mélange d'acides gras contenant au moins un acide gras insaturé, en particulier, un acide gras mono-insaturé, l'acide gras insaturé provenant d'huile végétale ou de graisse animale.

De préférence l'acide gras insaturé est l'acide oléique, l'acide linoléique, l'acide linolénique, ou leurs mélanges, en particulier, l'acide gras insaturé est l'acide oléique.

De préférence, les réactions de dimérisation et de trimérisation s'effectuent à partir d'un mélange d'acides gras d'huile de colza, d'huile de soja, d'huile de coton, d'huile de riz, d'huile de tournesol, d'huile de carthame, d'huile d'olive, d'huile de maïs, d'huile de raisin, de suif, d'huile de poisson, ou à partir d'une fraction particulière d'acide gras d'une huile ou d'une graisse, telle qu'une fraction d'acide oléique d'huile de palme.

Les réactifs peuvent comporter, en outre, un acide monocarboxylique.

Par acide monocarboxylique, on entend une chaîne hydrocarbonée comportant une seule fonction carboxylique, c'est-à-dire non substituée par une autre fonction ou un autre groupement qu'une fonction carboxylique (CO₂H).

De préférence, la teneur en acide monocarboxylique est inférieure ou égale à 20% en poids donné sur le poids total des réactifs. Plus préférentiellement, la teneur en acide monocarboxylique est inférieure à 10% en poids, encore plus préférentiellement inférieure à 5% en poids, en particulier inférieure à 2% en poids, les pourcentages en poids étant donnés sur le poids total des réactifs.

La présence d'acide monocarboxylique dans la réaction peut être due à la présence d'acide monocarboxylique (ou monomère d'acide) dans le mélange de trimère d'acide et de dimère d'acide, en tant que sous-produit de la réaction de trimérisation et/ou de dimérisation.

Dans ce cas, l'acide monocarboxylique peut être l'acide oléique, l'acide linoléique, l'acide linolénique, ou leurs mélanges.

L'acide monocarboxylique peut également être ajouté aux autres réactifs.

De préférence, l'acide monocarboxylique ainsi ajouté est saturé.

De préférence, l'acide monocarboxylique ajouté est un acide gras, en particulier saturé. De préférence il est choisi parmi le groupe constitué par l'acide laurique, l'acide myristique, l'acide palmitique, l'acide stéarique, l'acide béhénique et leurs mélanges.

De préférence, la teneur en polyol(s) mis en oeuvre dans le procédé d'obtention du polyester, est supérieure ou égale à 30% en poids, le pourcentage en poids étant donné sur le poids total des réactifs mis en réaction. Plus préférentiellement, la teneur en polyol(s) est supérieure ou égale à 35% en poids, encore plus préférentiellement, supérieure ou égale à 37% en poids, les pourcentages en poids étant donnés sur le poids total des réactifs mis en réaction.

De préférence, la teneur en polyol(s) est comprise entre 30% et 50% en poids, plus préférentiellement entre 35% et 45% en poids, les pourcentages en poids étant donnés sur le poids total des réactifs mis en réaction.

Avantageusement, le polyol est constitué de 66% à 74% en poids de 1,6-hexanediol et de 26% à 34% en poids de dimère de diol, les pourcentages en poids étant donnés sur le poids total de polyol.

De préférence, la teneur en acide dicarboxylique, hors dimère d'acide, est supérieure ou égale à 35% en poids, le pourcentage en poids étant donné sur le poids total des réactifs mis en réaction. Plus préférentiellement, la teneur en acide dicarboxylique, hors dimère d'acide, est supérieure ou égale à 37% en poids, encore plus préférentiellement supérieure ou égale à 40% en poids, les pourcentages en poids étant donnés sur le poids total des réactifs mis en réaction.

De préférence, la teneur en acide dicarboxylique est comprise entre 35% et 55% en poids, plus préférentiellement entre 37% et 50% en poids, les pourcentages en poids étant donnés sur le poids total des réactifs mis en réaction.

De préférence, la teneur en trimère d'acide est supérieure ou égale à 5% en poids, le pourcentage en poids étant donné sur le poids total des réactifs mis en réaction. Plus préférentiellement, la teneur en trimère d'acide est supérieure ou égale à 7% en poids, encore plus préférentiellement supérieure ou égale à 8% en poids, les pourcentages en poids étant donnés sur le poids total des réactifs mis en réaction.

De préférence, la teneur en trimère d'acide est comprise entre 5% et 25% en poids, plus préférentiellement entre 5% et 19% en poids, encore plus préférentiellement entre 7% et 17% en poids, les pourcentages en poids étant donnés sur le poids total des réactifs mis en réaction.

De préférence, la teneur en dimère d'acide est inférieure ou égale à 10% en poids, le pourcentage en poids étant donné sur le poids total des réactifs mis en réaction. Plus préférentiellement, la teneur en dimère d'acide est inférieure ou égale à 5% en poids, encore plus préférentiellement inférieure ou égale à 3% en poids, les pourcentages en poids étant donnés sur le poids total des réactifs mis en réaction. Généralement, la teneur en dimère d'acide est supérieure à 0,1 % en poids par rapport au poids total des réactifs mis en réaction.

Plus particulièrement, l'invention vise un polyester susceptible d'être obtenu par mise en réaction des réactifs suivants :
- au moins 30% en poids de polyol,
- au moins 35% en poids d'acide dicarboxylique autre qu'un dimère d'acide,
- au moins 5% en poids de trimère d'acide,
- au plus 10% en poids de dimère d'acide, et
- au plus 20% en poids d'acide monocarboxylique,
les pourcentages en poids étant donnés sur le poids total des composés mis en réaction.

Encore plus particulièrement, l'invention vise un polyester susceptible d'être obtenu par mise en réaction des réactifs suivants :
- au moins 35% en poids, préférentiellement au moins 37% en poids, de polyol
- au moins 37% en poids, préférentiellement au moins 40% en poids, d'acide dicarboxylique autre qu'un dimère d'acide,
- au moins 5% en poids, préférentiellement au moins 7% en poids, de trimère d'acide,
- au plus 10% en poids, préférentiellement au plus 5% en poids, de dimère d'acide, et
- au plus 20% en poids, préférentiellement au plus 15% en poids, d'acide monocarboxylique,
les pourcentages en poids étant donnés sur le poids total des composés mis en réaction.

Un polymère particulièrement préféré est obtenu à partir des gammes préférées mentionnées ci-avant.

La présente invention vise également un procédé de préparation du polyester selon l'invention.

Le procédé de préparation du polyester selon l'invention, comporte une étape de mise en réaction de plusieurs réactifs, les réactifs comportant un polyol, un acide dicarboxylique autre qu'un dimère d'acide, et au moins un trimère d'acide, en mélange ou non avec au moins un dimère d'acide, étant entendu que si un dimère d'acide est présent, le mélange de trimère d'acide et de dimère d'acide contient au moins 50% en poids de trimère d'acide, le pourcentage en poids étant donné sur le poids total dudit mélange, la teneur en trimère d'acide étant supérieure ou égale à 5% en poids, le pourcentage en poids étant donné sur le poids total des réactifs mis en réaction.

Plus particulièrement, le procédé de préparation du polyester selon l'invention comporte une étape de mise en réaction des réactifs, ceux-ci comportant au moins un polyol, un acide dicarboxylique autre qu'un dimère d'acide, et un mélange de trimère d'acide et de dimère d'acide contenant au moins 50% en poids de trimère d'acide, le pourcentage en poids étant donné sur le poids total du mélange ; de préférence, la teneur en trimère d'acide est supérieure ou égale à 5% en poids, le pourcentage en poids étant donné sur le poids total des réactifs mis en réaction.

Les réactifs sont tels que décrits ci-avant, y inclus les modes avantageux et préférentiels.

La mise en réaction des réactifs commence par une phase de solubilisation des réactifs qui s'effectue à chaud, de préférence entre 90 et 180°C. La phase de solubilisation peut s'effectuer par élévation constante de la température ou par palier de température, en fonction des températures de fusion des réactifs à solubiliser.

La température de réaction est comprise entre 185 et 220°C, préférentiellement entre 190 et 210°C.

De préférence, le procédé de préparation du polyester est effectué sous atmosphère inerte, en particulier sous azote.

La fin de réaction est contrôlée par la valeur de l'indice d'acide. La réaction est considérée terminée lorsque l'indice d'acide est inférieur à 15 mg KOH/g, préférentiellement, inférieur à 10 mg KOH/g. Avantageusement, en fin de réaction, l'indice d'acide est proche de 5 mg KOH/g, plus ou moins 2.

Le temps de réaction dure entre 3 et 20 heures, préférentiellement entre 4 et 18 heures.

Un catalyseur peut être utilisé dans le procédé de préparation du polyester selon l'invention. Typiquement, il s'agit d'un catalyseur d'estérification. A titre d'exemple, il peut s'agir de l'acide phosphorique, de l'acide hypophosphorique, de l'acide méthanesulfonique, de l'acétate de potassium, de l'hydroxyde de sodium ou de leurs mélanges. Avec catalyseur, le temps de réaction est diminué et dure entre 3 et 10 heures, préférentiellement entre 4 et 6 heures.

Avantageusement, le procédé de préparation du polyester selon l'invention est réalisé sans catalyseur. L'absence de catalyseur est un avantage économique. Outre l'économie sur le coût du catalyseur, il y a également une économie sur le coût du procédé, pour lequel il n'est pas nécessaire d'ajouter une étape de récupération du catalyseur, de type neutralisation et/ou filtration. Autre avantage, le polyester n'est pas souillé par la présence, même sous forme de trace, de catalyseur. En l'absence de catalyseur, le temps de réaction dure généralement entre 10 et 20 heures, préférentiellement entre 12 et 18 heures, plus préférentiellement, entre 13 et 16 heures.

Le polyester obtenu selon le procédé de préparation selon l'invention est solide. Le polyester peut être mis sous forme particulaire, telle que sous forme de flocon, de poudre ou de bille.

Le polyester selon l'invention est stable dans le temps et aux variations de température, comme cela est montré à l'Exemple 2 b).

L'invention concerne également une composition comprenant le polyester selon l'invention, et une matière grasse.

De préférence, la matière grasse est choisie parmi le groupe constitué par les monoglycérides, les diglycérides, les triglycérides, les alcools gras, les acides gras, les esters d'acides gras, leurs dérivés, tels que les diacides, les diesters, et des esters formés à partir d'au moins un acide gras et d'au moins un alcool ou d'au moins un polyol, ou d'un mélange d'au moins un alcool et d'au moins un polyol.

Plus préférentiellement, la matière grasse est choisie parmi le groupe constitué par les monoglycérides, les diglycérides, les triglycérides, les alcools gras, et les esters formés à partir d'au moins acide gras et d'au moins un alcool ou d'au moins un polyol, ou d'un mélange d'au moins un alcool et d'au moins un polyol.

Encore plus préférentiellement, la matière grasse est choisie parmi le groupe constitué par les triglycérides, les esters d'acides gras et leurs mélanges.

Selon un premier mode de réalisation de la composition selon l'invention, la teneur en polyester est supérieure ou égale à 40% en poids, de préférence au moins 50% en poids, les pourcentages en poids étant donnés sur le poids total en polyester et matière grasse (Exemple 3 a)).

La composition est alors sous forme d'une pâte ou d'un solide, selon que la matière grasse soit liquide ou solide à 25°C, plus ou moins 5°C, à pression atmosphérique.

Par pâte, on entend une composition visqueuse semi-solide.

La texture de la composition ainsi obtenue permet à celle-ci d'être broyée et d'obtenir une poudre.

Une telle composition comprenant une matière grasse et un polyester conforme à l'invention présente un point de fusion inférieur à celui du polyester seul (Exemple 4). Ceci permet alors de mettre oeuvre le polyester à des températures réduites, ce qui est particulièrement avantageux dans le domaine de la cosmétique par exemple, où il est d'usage de travailler à des températures inférieures à 80°C pour ne pas altérer des composés sensibles aux hautes températures.

Selon un second mode de réalisation de la composition selon l'invention, la teneur en polyester est inférieure à 40% en poids, de préférence inférieure ou égale à 35% en poids, plus préférentiellement inférieure ou égale à 30% en poids, les pourcentages en poids étant donnés par rapport au poids total en polyester et matière grasse (Exemple 3b)).

De préférence, la matière grasse est liquide à 40°C à pression atmosphérique.

De préférence, la matière grasse est liquide à 25°C, plus ou moins 5°C, à pression atmosphérique.

La composition comprenant la matière grasse et le polyester est alors sous forme d'un gel, c'est-à-dire, un réseau tridimensionnel d'un solide dans un liquide.

La composition selon l'invention selon le second mode de réalisation, est stable à la température, non collante, non filante, s'étale facilement et est brillante (Exemple 5). Cette composition, de par ses caractéristiques, peut avantageusement être utilisée dans le domaine des revêtements et de la cosmétique.

Les compositions selon l'invention présentent l'avantage de s'homogénéiser plus facilement avec d'autres matières grasses que le polyester seul.

Les compositions selon l'invention sont préparées selon un procédé comportant les étapes suivantes :
- la mise en contact du polyester et de la matière grasse, et
- le chauffage du polyester mis en contact avec la matière grasse à une température d'au moins 60°C.

Lorsque la composition est homogène et translucide, la composition est refroidie.

Avantageusement, le polyester, qui est solide, se présente sous une forme appropriée pour la mise en contact avec la matière grasse, telle que par exemple, sous une forme fractionnée, comme des flocons ou une poudre.

La mise en contact du polyester avec la matière grasse peut s'effectuer en une ou plusieurs fois, c'est-à-dire qu'une première mise en contact peut être réalisée avec une partie de la matière grasse, puis au moins une seconde mise en contact du polyester, qui a déjà été mis en contact avec la première partie de la matière grasse, est à nouveau mis en contact avec au moins une seconde partie de la matière grasse. Un tel ajout en plusieurs fois de la matière grasse permet en particulier d'ajuster la teneur en polyester dans la composition et/ou de modifier ou d'adapter la texture (solide, pâte, gel) de la composition à l'utilisation qui en sera faite. La matière grasse introduite lors de ces ajouts peut être identique ou différente. Comme indiqué ci-avant, la texture de la composition dépend également du point de fusion de la matière grasse. Le choix de la matière grasse permet donc également d'adapter la texture lors des différents ajouts.

Par exemple, selon un mode de réalisation particulier du procédé de préparation de la composition selon le second mode de réalisation, le procédé comporte les étapes suivantes :
- la mise en contact du polyester et d'une partie de la matière grasse,
- le chauffage du polyester mis en contact avec la matière grasse à une température d'au moins 60°C,
- le refroidissement de la composition ainsi obtenue, qui est une composition selon le premier mode de réalisation, puis
- la mise en contact du polyester présent dans la composition selon le premier mode de réalisation et d'une autre partie de la matière grasse,
- le chauffage du polyester mis en contact avec la matière grasse à une température d'au moins 60°C,
- le refroidissement de la composition ainsi obtenue, qui est une composition selon le second mode de réalisation.

Ainsi, la composition selon le premier mode de réalisation, sous forme solide ou pâteuse, peut être utilisée pour préparer la composition selon le second mode de réalisation, sous forme de gel.

Préférentiellement, et quel que soit le mode de réalisation du procédé, la température de chauffage est comprise entre 60°C et 100°C, plus préférentiellement encore entre 70°C et 90°C.

Avantageusement, le chauffage est réalisé sous agitation.

La présente invention divulgue également une utilisation du polyester selon l'invention ou de la composition selon l'invention en tant qu'épaississant.

Le polyester selon l'invention augmente la viscosité des matières grasses liquides à 25°C +/- 5°C à pression atmosphérique, avec lesquelles il est compatible. Par compatible, on entend apte à se mélanger de telle sorte à former une seule phase homogène. Les compositions selon l'invention ont en effet une viscosité plus importante que la viscosité de la matière grasse. Le polyester selon l'invention est viscosifiant ou épaississant. L'Exemple 6 donne des exemples d'accroissement de viscosité observé avec des compositions selon l'invention.

Un tel polyester ou une telle composition peut trouver des applications dans le domaine des lubrifiants pour contrôler la viscosité des esters ou huiles utilisés dans ce domaine, mais également dans le domaine pétrolier, où la composition selon l'invention peut remplacer des cires, des paraffines ou des graisses.

Le polyester selon l'invention est également apte à modifier les propriétés rhéologiques d'une matière grasse. Il a un effet rhéofluidifiant et thixotrope.

Par rhéofluidifiant, on entend un composé ou un mélange de composé, dont la viscosité diminue lorsqu'il est soumis à une contrainte croissante, telle qu'un cisaillement.

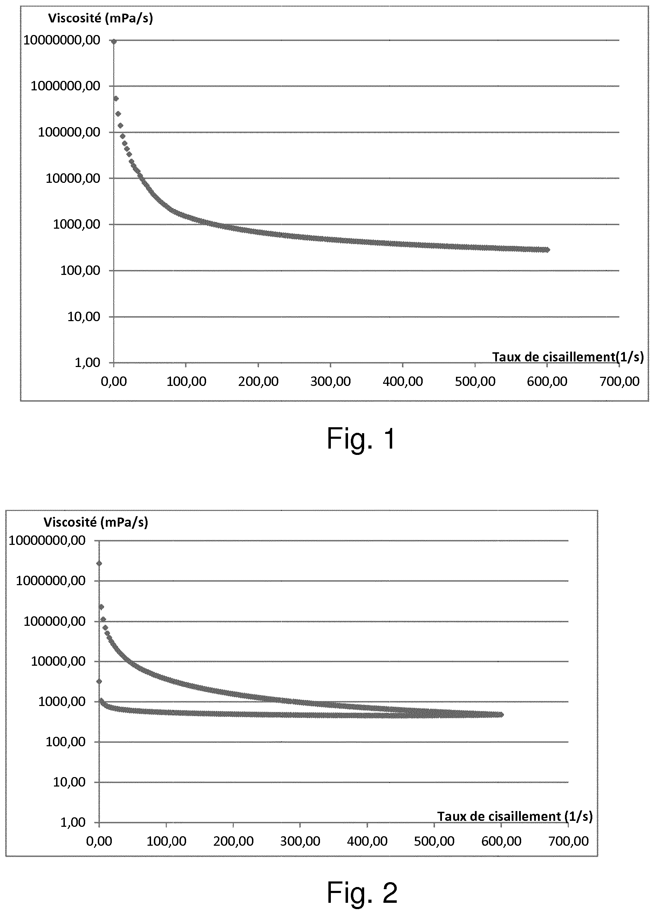

Comme le montre la courbe d'écoulement (Exemple 7, Fig. 1), plus le taux de cisaillement augmente, plus la viscosité de la composition selon l'invention diminue. Le polyester de l'invention rhéofluidifie la matière grasse.

Le polyester de l'invention a également un effet thixotrope, comme le montre la courbe d'écoulement (Exemple 7, Fig. 2). La composition selon l'invention retrouve sa texture gélifiée après avoir subi un cisaillement. La composition selon l'invention est thixotrope.

Le polyester pourra avantageusement être utilisé dans des produits cosmétiques en tant qu'épaississant rhéofluidifiant thixotrope.

La présente invention concerne également l'utilisation du polyester selon l'invention ou de la composition selon l'invention, en tant que rhéofluidifiant.

Il peut, en effet, être intéressant d'ajouter le polyester ou la composition selon l'invention à des matières grasses solides à température ambiante, afin d'apporter ou d'améliorer la propriété rhéofluidifiante de la matière grasse.

La présente invention vise également une émulsion comprenant le polyester selon l'invention ou la composition selon l'invention, et de l'eau.

Une émulsion est généralement obtenue à partir d'une phase aqueuse et d'une phase huileuse. Dans la présente demande, la phase huileuse comprend le polyester selon l'invention ou la composition selon l'invention.

L'émulsion comprend en outre au moins un tensioactif. Le tensioactif est soit compris dans la phase huileuse, soit compris dans la phase aqueuse. Selon le type d'émulsion souhaitée, eau dans huile ou huile dans eau, le tensioactif aura plus d'affinité à se solubiliser dans la phase continue, respectivement la phase huileuse ou la phase aqueuse.

Le tensioactif peut être une matière grasse telle que définie dans la présente demande. A titre d'exemple, il peut s'agir d'esters de sorbitan, des esters de glycérol, des esters de polyglycérol, des esters de sucrose, des alkylpolyglucosides, ou des alkylpolypentosides.

Un procédé de préparation d'une émulsion selon l'invention comporte une étape de chauffage à une température comprise entre 60°C et 100°C, de préférence entre 70°C et 90°C, de la composition selon l'invention et de l'eau, et une étape de mélange de la composition avec l'eau.

Pour une émulsion eau dans huile, l'eau est additionnée à la composition sous agitation.

Pour une émulsion huile dans eau, la composition est additionnée à l'eau sous agitation.

Lorsque la composition est un constituant d'une phase huileuse et/ou l'eau est un constituant d'une phase aqueuse, il y a une étape préalable de mélange des constituants de la phase huileuse et/ou des constituants de la phase aqueuse.

La phase huileuse ou la composition, et la phase aqueuse ou l'eau, sont chauffées chacune à une température comprise entre 60°C et 100°C, de préférence entre 70°C et 90°C. Puis la phase huileuse ou la composition est mélangée à la phase aqueuse ou à l'eau, ou la phase aqueuse.

Pour une émulsion eau dans huile, l'eau ou la phase aqueuse est additionnée à la composition ou la phase huileuse sous agitation.

Pour une émulsion huile dans eau, la composition ou la phase huileuse est additionnée à l'eau ou la phase aqueuse sous agitation.

La phase huileuse peut comprendre, outre la composition comprenant de la matière grasse et le polyester, un tensioactif.

De préférence, l'émulsion est de type eau dans huile. La composition ou la phase huileuse comprend au moins un tensioactif.

Le polyester est de préférence présent en des quantités allant de 0,1% à 15% en poids, préférentiellement de 1% à 10% en poids, plus préférentiellement de 2% à 6% en poids, le pourcentage étant donné sur le poids total de l'émulsion.

Avantageusement, l'émulsion selon l'invention est utilisée en cosmétique.

La phase huileuse d'une émulsion cosmétique peut contenir des ingrédients solubles dans une phase huileuse, tels qu'un émollient, une cire, un actif, un agent anti-UV et/ou une vitamine.

La phase aqueuse d'une émulsion cosmétique contient des ingrédients solubles dans une phase aqueuse, tel qu'un humectant, un agent stabilisant, un conservateur.

De façon inattendue, l'émulsion selon l'invention présente une viscosité élevée. Des tests comparatifs (Exemple 8 b)) montrent que les émulsions contenant le polyester selon l'invention, voient leur viscosité dynamique à 25 °C croître de manière plus importante qu'avec d'autres polyesters.

Ce fort pouvoir épaississant sur des émulsions, fait du polyester, ou de la composition selon l'invention, un bon stabilisant d'émulsion.

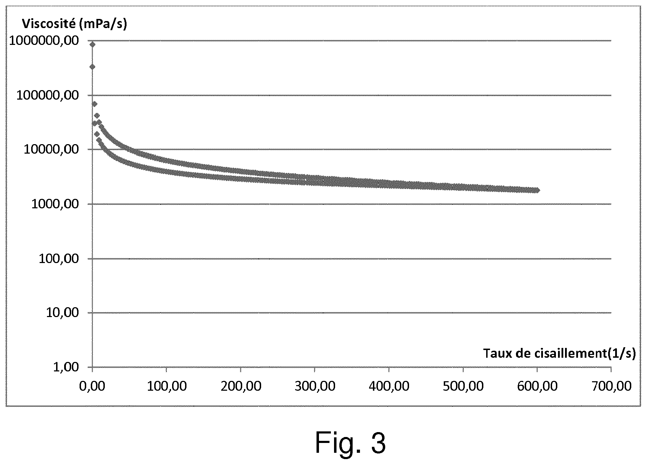

Avantageusement, l'émulsion selon l'invention présente également de bonnes propriétés de rhéfluidification et de thixotropie (Exemple 10, Fig. 3). Le rhéogramme (Fig. 3) montre qu'après diminution de la viscosité lors du cisaillement, la viscosité croît à nouveau dès lors que le cisaillement s'arrête. On peut en conclure que le polyester rend l'émulsion thixotrope.

La présente demande divulgue également l'utilisation du polyester l'invention, ou la composition selon l'invention, ou l'émulsion selon l'invention, dans des produits cosmétiques.

Enfin, la présente demande divulgue un produit cosmétique comprenant le polyester selon l'invention, ou une composition d'un tel polyester avec une matière grasse selon l'invention, ou l'émulsion selon l'invention, et un principe actif et/ou un pigment ou un colorant.

Par produit cosmétique, on entend un mélange de composés destiné à être mis en contact avec les parties superficielles du corps humain (l'épiderme, les systèmes pileux et capillaire, les ongles, les lèvres et les organes génitaux externes) ou avec les dents et les muqueuses buccales, en vue, exclusivement ou principalement, de les nettoyer, de les parfumer, d'en modifier l'aspect, de les protéger, de les maintenir en bon état ou de corriger les odeurs corporelles. A titre d'exemple de produit cosmétique, on peut citer les crèmes hydratantes, telle que les crèmes de nuit, les rouge à lèvres, les gloss à lèvres, les baumes à lèvres, les crayons à sourcils, les eyeliners, les ombres à paupières, les mascaras, les fonds de teint, les lotions, les soins pour cheveux sous forme de gels, les produits solaires, et les gels douche.

Le produit cosmétique peut comprendre en outre au moins un adjuvant, tels qu'un conservateur, un opacifiant, un abrasif, un parfum, un ajusteur de pH, un stabilisateur de mousse, un humectant, un filmogène, un bactéricide, et/ou un agent tannant.

D'autres caractéristiques et avantages de l'invention apparaîtront dans les exemples qui suivent, donnés à titre illustratif, et avec référence aux figures :
- La Figure 1 représente une courbe d'écoulement d'une composition selon l'invention, comprenant le polyester selon l'invention et du triglycéride caprylique/caprique dans un rapport 15/85,
- La Figure 2 représente l'évolution de la viscosité d'une composition comprenant le polyester selon l'invention et du triglycéride caprylique/caprique dans un rapport 15/85, en fonction de l'application d'un taux de cisaillement puis de l'arrêt de cette contrainte,
- La Figure 3 représente l'évolution de la viscosité d'une émulsion selon l'invention en fonction de l'application d'un taux de cisaillement puis de l'arrêt de cette contrainte.

### Exemple 1 : Procédé de préparation d'un polyester selon l'invention

Dans un réacteur muni d'un thermocouple, d'un agitateur mécanique et d'un réfrigérant, sont introduits du 1,6-hexanediol (215,1 g), un dimère de diol résultant de la dimérisation de l'acide oléique suivie d'une hydrogénation du dimère de l'acide oléique (le dimère de diol comporte 36 atomes de carbone) (87,5 g), de l'acide sébacique (331,6 g) et un mélange de trimère d'acide et de dimère d'acide (116 g), ce mélange contenant trois constituants, 89% en poids de trimère d'acide, 10,9% en poids de dimère d'acide et 0,1% en poids de monomère d'acide, les pourcentages en poids étant donnés sur le poids total des trois constituants du mélange.

Le mélange de trimère d'acide et de dimère d'acide a été obtenu par dimérisation et trimérisation d'acides gras d'huile de colza contenant au moins 80% en poids d'acides gras comportant 18 atomes de carbone, le pourcentage étant donné sur le poids total d'acides gras. Le mélange obtenu a été distillé afin de concentrer le distillat en trimère d'acide et obtenir le mélange de trois constituants utilisé dans cet exemple.

Sous atmosphère d'azote, le mélange est chauffé à 100°C, l'agitation est mise en route et le mélange est porté à 130°C. Une fois que l'acide sébacique est totalement dissout, le mélange est porté à 160°C.

Lorsque l'eau commence à être collectée, la température du milieu réactionnel est portée à 200°C. Après 15 heures à cette dernière température, l'indice d'acide atteint 6,77 mg KOH/g.

Le chauffage est alors stoppé et, lorsque le mélange atteint 150°C, il est versé sur une plaque en aluminium pour réaliser un film fin. Une fois le film refroidi, le polyester est solide, de couleur légèrement jaune. Le polyester est cassé sous forme de flocons.

### Exemple 2 : Caractérisation du polyester selon l'invention

### a) Température de fusion

La fourchette de température de fusion du polyester préparé à l'Exemple 1 a été déterminée par la technique de calorimétrie à balayage différentiel (DSC), à l'aide d'un appareil Perkin Elmer DSC 800.

Un creuset en aluminium fermé a été utilisé sous atmosphère d'azote. La température passe de -50°C à 95°C avec une augmentation de 10°C par minute et de 95°C à -50°C avec une diminution de 20°C par minute. Après 4 minutes à -50°C, la température est à nouveau portée à 95°C à une vitesse de 5°C par minute.

La courbe obtenue montre que le polyester commence à fondre à 44,79°C et que la température du sommet du pic de fusion est de 51,35°C.

### b) Stabilité

### Test 1 : stabilité dans le temps à la température

Le polyester a été soumis à un chauffage. A 40°C, le polyester préparé à l'Exemple 1 commence à se ramollir et à 60°C, est observé un mélange de polyester fondu et de polyester solide.

Après 5 jours à 60°C, aucune différence n'est observée. De retour à température ambiante, le polyester a le même aspect qu'avant le test, c'est à dire un solide de même couleur légèrement jaune.

### Test 2 : stabilité à la température

Pour ce test, la technique de calorimétrie à balayage différentiel (DSC) a été utilisée avec le même appareil que dans l'Exemple 2 a). Un creuset en aluminium fermé a été utilisé. Sous atmosphère d'azote, la température passe de -20°C à 100°C avec une augmentation de 10°C par minute et de 100°C à -20°C avec une diminution de 10°C par minute. Le polyester préparé à l'Exemple 1 a été ainsi soumis à cinq cycles de chauffage à 100°C suivi d'un refroidissement à -20°C. Les mêmes températures de fusion (à 1,5°C près) que celle observées à l'Exemple 2 a) ont été trouvées après ces cinq cycles.

### Exemple 3: Procédé de préparation de compositions selon l'invention

### a) Composition à texture pâteuse (premier mode de réalisation)

Un triglycéride substitué par des chaînes hydrocarbonées comportant 8 et 10 atomes de carbone est ajouté au polyester préparé à l'Exemple 1 dans un rapport massique triglycéride/polyester = 40/60. L'ensemble est chauffé à 80°C et agité jusqu'à obtenir un mélange translucide et homogène. La composition obtenue est laissée refroidir à température ambiante. Une pâte légèrement jaune est obtenue.

### b) Composition à texture gélifiée (second mode de réalisation)

Un triglycéride substitué par des chaînes hydrocarbonées comportant 8 ou10 atomes de carbone est ajouté au polyester préparé à l'Exemple 1 dans un rapport massique triglycéride/polyester = 85/15. L'ensemble est chauffé à 80°C et agité jusqu'à obtenir un mélange translucide et homogène. La composition obtenue est laissée refroidir à température ambiante. Un gel légèrement jaune est alors obtenu.

L'évaluation des caractéristiques des compositions a été réalisée le jour suivant pour laisser le temps à la matière grasse de se viscosifier sous l'effet du polyester.

### Exemple 4 : Caractérisation d'une composition selon l'invention (premier mode de réalisation)

### Température de fusion

Selon le protocole décrit à l'Exemple 2 a), il a été observé que la composition à texture pâteuse préparée à l'Exemple 3 a) commençait à fondre à 37,00°C et que la température du sommet du pic de fusion était de 43,50°C.

La température de fusion de cette composition est donc inférieure à la température du polyester seul. Ainsi, la manipulation du polyester, lorsqu'il est en mélange avec de la matière grasse, est facilitée, puisqu'elle celle-ci peut se faire à des températures plus basses.

### Exemple 5 : Caractérisation d'une composition selon l'invention (second mode de réalisation) gel de l'Exemple 3b)

### - Stabilité

La composition à texture gélifiée de l'Exemple 3 b) a été soumise à un chauffage : à 40°C, la composition commence à se liquéfier et à 60°C, elle est complètement liquide.

Après 5 jours à 60°C, la composition est ramenée à une température ambiante (soit 25°C + /- 5°C) et retrouve sa texture gélifiée après 1 jour.

### - Caractère non collant

L'évaluation de cette caractéristique est réalisée en appliquant de la composition à texture gélifiée sur deux doigts, par exemple sur l'index et le pouce. Juste après l'application et après 2 minutes, les deux doigts sont pressés puis séparés. Aucune force n'est nécessaire pour séparer les deux doigts, la composition est donc non collante.

### - Caractère non filant

L'évaluation de cette caractéristique est réalisée en compressant de la composition à texture gélifiée entre deux doigts, le pouce et l'index par exemple, et en séparant les doigts, trois fois de suite. Aucun fil ne se forme lorsque les doigts s'écartent, la composition est donc non filante.

### - Présence de pics

Selon le protocole précédent, lorsque les doigts se séparent, aucun pic n'est formé.

### - Facilité d'étalement

La composition s'étale facilement sur la peau dès le premier mouvement circulaire effectué par un doigt, par exemple l'index, sur la peau. Après trois mouvements circulaires, la composition forme une fine couche sur la peau.

### - Brillance

En approchant la composition d'une source lumineuse, les rayons sont réfléchis et la surface de la composition apparaît brillante.

### Exemple 6 : Effet épaississant du polyester selon l'invention

La viscosité de la composition gélifiée selon l'Exemple 3 b) a été mesurée, ainsi que la viscosité d'autres compositions obtenues à partir du polyester de l'Exemple 1 et selon le procédé de préparation décrit à l'Exemple 3 b). La ou les matière(s) grasse(s) et ratio(s) massiques sont indiqués dans le Tableau 1.

Pour cette mesure, un rhéomètre Anton Paar MCR 102 a été utilisé avec les caractéristiques suivantes :
- plan sablé de 25mm de diamètre,
- cône CP25-2, avec un diamètre de 24,97 mm, un angle de 1,982°, une troncature de 103 µm.

Pour chaque composition, la viscosité a été mesurée lorsque la composition est soumise à 25°C à un faible taux de cisaillement en échelle logarithmique (valeurs proches de zéro correspondant au plateau Newtonien), c'est-à-dire pour lequel la viscosité est alors constante. Les résultats sont regroupés dans le Tableau 1.

**Tableau 1**

| **Compositions : Polyester / matière(s) grasse(s) = 15/85** | **Viscosité (mPa/s)** | **Accroissement de viscosité** |
|---|---|---|
| Polyester + triglycérides à chaînes hydrocarbonées en C8 et C10 (15/85) | 4,7x10⁸ | 1,68x10⁷ |
| Polyester + laurate d'isoamyle (15/85) | 6,1x10⁸ | 1,01x10⁸ |
| Polyester + huile de tournesol (15/85) | 9,9x10⁷ | 1,62x10⁶ |
| Polyester + triglycérides à chaînes hydrocarbonées en C8 et C10 + laurate d'isoamyle (15/10/75 = 15/85) | 1,4x10⁹ | 2,33x10⁸ |

L'accroissement de viscosité est calculé en comparant la viscosité de la composition par rapport à la viscosité de la matière grasse.

### Exemple 7 : Effets rhéofluidifiant et thixotrope du polyester dans une composition selon l'invention

### - Rhéofluidifiant

Pour ce test, un rhéomètre Anton Paar MCR 102, équipé d'un plan sablé de 25mm de diamètre, et d'un cône CP25-2, avec un diamètre de 24,97 mm, un angle de 1,982° et une troncature de 103 µm, a été utilisé. La composition préparée à l'Exemple 3 b) a subi à 20°C, un taux de cisaillement correspondant à une augmentation de 0 à 600 s⁻¹. La courbe d'écoulement obtenue est représentée en Figure 1. On peut y observer que, plus le taux de cisaillement augmente, plus la viscosité de la composition diminue. La composition est donc rhéofluidifiante grâce à la présence du polyester selon l'invention.

### - Thixotrope

Pour ce test, le même rhéomètre que celui décrit ci-dessus a été utilisé. La composition préparée à l'Exemple 3 b) a subi, à 20°C, un taux de cisaillement correspondant à une augmentation de 0 à 600 s⁻¹, suivi d'une diminution de ce taux de cisaillement de 600 à 0 s⁻¹. Le rhéogramme établi (Fig. 2) est une courbe traduisant l'évolution de la viscosité en fonction du cisaillement. On peut observer que le chemin de la courbe tracé au retour se situe nettement en dessous de celui établi à l'aller, et que la composition voit sa viscosité s'accroître à nouveau dès lors que le cisaillement s'arrête. On peut en conclure que, grâce à la présence du polyester selon l'invention dans la composition selon l'invention, la composition est thixotrope.

### Exemple 8 : Procédé de préparation d'une émulsion selon l'invention

Différentes émulsions A à E, à texture gélifiée ont été préparées selon le protocole général décrit ci-dessous. Les composés et quantités (pourcentages massiques) utilisés pour préparer ces émulsions sont indiqués dans le Tableau 2 ci-dessous.

La phase huileuse est préparée en mélangeant un tensioactif, à savoir un mélange de polyricinoléate de polyglycérol et d'isostéarate de sorbitan, avec du diheptanoate de monopropylène glycol, du laurate d'isoamyle, du triglycéride caprylique/caprique et de la triéthylhexanoin. Le polyester de l'Exemple 1 est ensuite ajouté aux autres composés.

La phase huileuse est alors chauffée à 80°C jusqu'à ce que le polyester soit complètement fondu et que la phase soit homogène.

Parallèlement, la phase aqueuse est préparée dans un autre réacteur, en mélangeant de la glycérine et du sulfate de magnésium heptahydrate, et complétée avec de l'eau, puis en chauffant à 80°C.

Alors que la phase huileuse est agitée par un agitateur Rayneri tripale à une vitesse telle qu'un vortex se forme, la phase aqueuse est alors ajoutée lentement à la phase huileuse, selon un goutte à goutte ou un écoulement continu. De préférence, l'ajout tombe au centre du vortex.

Une fois toute la phase aqueuse ajoutée, l'émulsion est homogénéisée à l'aide d'un rotor/stator Ultra-turrax, à une vitesse de 15000 t/min.

L'émulsion est alors refroidie à température ambiante sous agitation à l'aide d'un mélangeur Rayneri.

A 30°C, un conservateur, du phénoxyéthanol, est ajouté.

**Tableau 2**

| | **Noms INCI (International Nomenclature of Cosmetic)** | **fonctions** | **A** | **B** | **C** | **D** | **E** |
|---|---|---|---|---|---|---|---|
| Phase huileuse | Polyricinoleate de polyglycerol et isostéarate de sorbitan (Radiamuls ® 7887 de Oleon) | tensioactif | 3* | 3 | 3 | 3 | 3 |
| | Diheptanoate de monopropylène glycol (Radia ® 7202 de Oleon) | émollient | 8 | 8 | 8 | 8 | 8 |
| | Laurate d'isoamyle (Radia ® 7750 de Oleon) | émollient | 4 | 4 | 4 | 4 | 4 |
| | Triglycéride caprylique / caprique (Radia ® 7104 de Oleon) | émollient | 10 | 8 | 6 | 4 | 0 |
| | Triéthylhexanoin (Radia ® | émollient | 8 | 8 | 8 | 8 | 8 |
| | 7610 de Oleon) | | | | | | |
| | Polyester de l'Exemple 1 | épaississant rhéologique | 0 | 2 | 4 | 6 | 10 |
| Phase aqueuse | Glycérine (Radia ® 4810 de Oleon) | humectant | 4 | 4 | 4 | 4 | 4 |
| | Eau | - | 62 | 62 | 62 | 62 | 62 |
| | Sulfate de magnésium heptahydrate (de Labosi) | agent stabilisant | 0,5 | 0,5 | 0,5 | 0,5 | 0,5 |
| | Phénoxyéthanol (Microcare ® PE de Thor) | conservateur | 0,5 | 0,5 | 0,5 | 0,5 | 0,5 |

| | | | | | | | |
|---|---|---|---|---|---|---|---|
| *les quantités sont exprimées en pourcentage en poids sur le poids total de l'émulsion | | | | | | | |

### Exemple 9 : Effet épaississant du polyester selon l'invention sur une émulsion

### a) Augmentation de la viscosité avec la teneur en polyester

Les viscosités, mesurées selon la méthode décrite à l'Exemple 6, des différentes émulsions A à E du Tableau 2, sont rassemblées dans le Tableau 3 ci-dessous.

**Tableau 3**

| | **A** | **B** | **C** | **D** | **E** |
|---|---|---|---|---|---|
| **Viscosités (Pa.s) à 25°C** | 0,6 | 4,0 | 38,8 | 93,8 | 694,0 |
| **Accroissement de viscosité** | - | 6,7 | 64,6 | 156,3 | 1156,6 |

Plus la teneur en polyester est élevée, plus l'émulsion présente une forte viscosité. Le polyester est viscosifiant ou épaississant même en faible quantité. Avec 2% en poids de polyester, la viscosité de l'émulsion est multipliée par 6,7.

Pour cette raison, le polyester peut être utilisé comme stabilisant et aider le formulateur à obtenir la viscosité souhaitée.

### b) Supériorité du polyester selon l'invention en tant qu'épaississant

Quatre émulsions ont été préparées : une émulsion de référence (Réf) ne contient pas d'épaississant, les émulsions F et G contiennent chacune un polyester épaississant Syncrowax commercialisé par Croda et l'émulsion H contient le polyester préparé à l'Exemple 1 (émulsion selon l'invention).

Les composés et quantités (pourcentages massiques) utilisés pour préparer ces émulsions sont indiqués dans le Tableau 4 ci-dessous. Ces émulsions ont par ailleurs été préparées selon le protocole décrit à l'Exemple 8.

**Tableau 4**

| | **Noms INCI (International Nomenclature of Cosmetic)** | **Réf** | **F** | **G** | **H** |
|---|---|---|---|---|---|
| Phase grasse | Polyglycérol Polyricinoléate et isostéarate de sorbitan (Radiamuls ® 7887 de Oleon) | 4 | 4 | 4 | 4 |
| | Propylène glycol dicaprilate/dicaprate (Radia ® 7207 de Oleon) | 2 | 2 | 2 | 2 |
| | laurate d'isoamyle (Radia ® 7750 de Oleon) | 10 | 10 | 10 | 10 |
| | caprylic / capric triglycéride (Radia ® 7104 de Oleon) | 8 | 4 | 4 | 4 |
| | copolymère béhénate de sorbitol/acide sébacique (Syncrowax ORM de Croda ®) | 0 | 4 | 0 | 0 |
| | Tribehenin et copolymère béhénate de sorbitol/acide sébacique (Syncrowax OSW de Croda ®) | 0 | 0 | 4 | 0 |
| | Polyester de l'Exemple 1 | 0 | 0 | 0 | 4 |
| Phase aqueuse | glycérine (Radia ® 4810 de Oleon) | 3 | 3 | 3 | 3 |
| | aqua | 71,8 | 71,8 | 71,8 | 71,8 |
| | Sulfate de magnésium heptahydrate (de Labosi) | 0,5 | 0,5 | 0,5 | 0,5 |
| | Phénoxyéthanol (Microcare ® PE de Thor) | 0,7 | 0,7 | 0,7 | 0,7 |

| | | | | | |
|---|---|---|---|---|---|
| *les quantités sont exprimées en pourcentage en poids sur le poids total de l'émulsion | | | | | |

Les viscosités de ces émulsions ont été mesurées selon la méthode décrite à l'Exemple 6, et sont rassemblées dans le Tableau 5 ci-dessous.

**Tableau 5**

| | **Réf** | **F** | **G** | **H** |
|---|---|---|---|---|
| **Viscosités (Pa.s) à 25°C** | 7,6 | 68,6 | 38,8 | 98,8 |
| **Accroissement de viscosité** | - | 9,0 | 5,1 | 13,0 |

Pour une même quantité de polyester épaississant (4% en poids sur le poids total de l'émulsion), la viscosité de l'émulsion est plus importante avec le polyester selon l'invention qu'avec les polyesters de Croda.

Des viscosités plus élevées ont pu être constatées avec d'autres polymères selon l'invention.

### Exemple 10 : Effets rhéofluidifiant et thixotrope du polyester dans une émulsion selon l'invention

Pour ce test, le même rhéomètre que celui décrit à l'Exemple 7 a été utilisé. L'émulsion H préparée à l'Exemple 9 b) a subi à 20°C, un taux de cisaillement correspondant à une augmentation de 0 à 600 s⁻¹, suivi d'une diminution de ce taux de cisaillement de 600 à 0 s⁻¹. Le rhéogramme établi (Fig. 3) est une courbe traduisant l'évolution de la viscosité en fonction du cisaillement. On peut observer que, plus le taux de cisaillement augmente, plus la viscosité de la composition diminue (courbe descente supérieure), puis que le chemin de la courbe tracé au retour se situe nettement en dessous de celui établi à l'aller, et que la composition voit sa viscosité s'accroître à nouveau dès lors que le cisaillement s'arrête. On peut en conclure que, grâce à la présence du polyester selon l'invention dans la composition selon l'invention, l'émulsion est rhéofluidifiante et thixotrope.

## Revendications

1. Polyester susceptible d'être obtenu par un procédé comportant une étape de mise en réaction de plusieurs réactifs, les réactifs comportant :
- au moins un polyol,
- au moins un acide dicarboxylique autre qu'un dimère d'acide, et
- au moins un trimère d'acide, en mélange ou non avec au moins un dimère d'acide, étant entendu que si un dimère d'acide est présent, le mélange de trimère d'acide et de dimère d'acide contient au moins 50% en poids de trimère d'acide, le pourcentage en poids étant donné sur le poids total dudit mélange,
la teneur en trimère d'acide étant supérieure ou égale à 5% en poids, le pourcentage en poids étant donné sur le poids total des réactifs mis en réaction.

2. Polyester selon la revendication 1, **caractérisé en ce que** la teneur en polyol est supérieure ou égale à 30% en poids, le pourcentage en poids étant donné sur le poids total des réactifs mis en réaction.

3. Polyester selon l'une quelconque des revendications précédentes, dans lequel la teneur en acide dicarboxylique, hors dimère d'acide, est supérieure ou égale à 35% en poids, le pourcentage en poids étant donné sur le poids total des réactifs mis en réaction.

4. Procédé de préparation d'un polyester, comportant une étape de mise en réaction de plusieurs réactifs, les réactifs comportant un polyol, un acide dicarboxylique autre qu'un dimère d'acide, et au moins un trimère d'acide, en mélange ou non avec au moins un dimère d'acide, étant entendu que si un dimère d'acide est présent, le mélange de trimère d'acide et de dimère d'acide contient au moins 50% en poids de trimère d'acide, le pourcentage en poids étant donné sur le poids total dudit mélange, la teneur en trimère d'acide étant supérieure ou égale à 5% en poids, le pourcentage en poids étant donné sur le poids total des réactifs mis en réaction.

5. Procédé de préparation d'un polyester, comportant une étape de mise en réaction de plusieurs réactifs, les réactifs comportant un polyol, un acide dicarboxylique autre qu'un dimère d'acide, et un mélange de trimère d'acide et de dimère d'acide contenant au moins 50% en poids de trimère d'acide, le pourcentage en poids étant donné sur le poids total du mélange.

6. Procédé selon la revendication 4 ou 5, réalisé sans catalyseur.

7. Composition comprenant le polyester selon l'une quelconque des revendications 1 à 3, et une matière grasse.

8. Utilisation du polyester selon l'une quelconque des revendications 1 à 3 ou de la composition selon la revendication 7, en tant qu'épaississant.

9. Utilisation du polyester selon l'une quelconque des revendications 1 à 3 ou de la composition selon la revendication 7, en tant que rhéofluidifiant.

10. Emulsion comprenant le polyester selon l'une quelconque des revendications 1 à 3 ou la composition selon la revendication 7, et de l'eau.

11. Utilisation du polyester selon l'une quelconque des revendications 1 à 3, ou la composition selon la revendication 7, ou l'émulsion selon la revendication 10, dans des produits cosmétiques.

12. Produit cosmétique comprenant le polyester selon l'une quelconque des revendications 1 à 3, ou la composition selon la revendication 7, ou l'émulsion selon la revendication 10, et un principe actif et/ou un pigment ou un colorant.

## Patentansprüche

1. Polyester, der durch ein Verfahren erhalten werden kann, das einen Schritt des Reaktionsumsetzens mehrerer Reagenzien umfasst, wobei die Reagenzien umfassen:
- mindestens ein Polyol,
- mindestens eine andere Dicarbonsäure als Säuredimer, und
- mindestens ein Säuretrimer in Mischung oder nicht mit mindestens einem Säuredimer, wobei, wenn ein Säuredimer vorhanden ist, die Mischung aus Säuretrimer und Säuredimer mindestens 50 Gew.-% Säuretrimer enthält, wobei der Gewichtsprozentanteil in Anbetracht des Gesamtgewichts der Mischung angegeben ist,
wobei der Gehalt an Säuretrimer höher oder gleich 5 Gew-% ist, wobei der Gewichtsprozentanteil in Anbetracht des Gesamtgewichts der reaktionsumgesetzten Reagenzien angegeben ist.

2. Polyester nach Anspruch 1, **dadurch gekennzeichnet, dass** der Gehalt an Polyol höher oder gleich 30 Gew.% ist, wobei der Gewichtsprozentanteil in Anbetracht des Gesamtgewichts der reaktionsumgesetzten Reagenzien angegeben ist.

3. Polyester nach einem der vorhergehenden Ansprüche, in dem der Gehalt an Dicarbonsäure, außer Säuredimer, höher oder gleich 35 Gew.-% ist, wobei der Gewichtsprozentanteil in Anbetracht des Gesamtgewichts der reaktionsumgesetzten Reagenzien angegeben ist.

4. Verfahren zur Herstellung eines Polyesters, umfassend einen Schritt des Reaktionsumsetzens mehrerer Reagenzien, wobei die Reagenzien ein Polyol, eine andere Dicarbonsäure als Säuredimer und mindestens ein Säuretrimer in Mischung oder nicht mit mindestens einem Säuredimer umfassen, wobei, wenn ein Säuredimer vorhanden ist, die Mischung aus Säuretrimer und Säuredimer mindestens 50 Gew.-% Säuretrimer enthält, wobei der Gewichtsprozentanteil in Anbetracht des Gesamtgewichts der Mischung angegeben ist, wobei der Gehalt an Säuretrimer höher oder gleich 5 Gew.-% ist, wobei der Gewichtsprozentanteil in Anbetracht des Gesamtgewichts der reaktionsumgesetzten Reagenzien angegeben ist.

5. Verfahren zur Herstellung eines Polyesters, umfassend einen Schritt des Reaktionsumsetzens mehrerer Reagenzien, wobei die Reagenzien ein Polyol, eine andere Dicarbonsäure als Säuredimer und eine Mischung aus Säuretrimer und Säuredimer umfassen, die mindestens 50 Gew.-% Säuretrimer enthält, wobei der Gewichtsprozentanteil in Anbetracht des Gesamtgewichts der Mischung angegeben ist.

6. Verfahren nach Anspruch 4 oder 5, das ohne Katalysator erfolgt.

7. Zusammensetzung, den Polyester nach einem der Ansprüche 1 bis 3 und ein Fett umfassend.

8. Verwendung des Polyesters nach einem der Ansprüche 1 bis 3 oder der Zusammensetzung nach Anspruch 7 als Verdickungsmittel.

9. Verwendung des Polyesters nach einem der Ansprüche 1 bis 3 oder der Zusammensetzung nach Anspruch 7 als Scherviskosemittel.

10. Emulsion, umfassend den Polyester nach einem der Ansprüche 1 bis 3 oder die Zusammensetzung nach Anspruch 7 und Wasser.

11. Verwendung des Polyesters nach einem der Ansprüche 1 bis 3 oder der Zusammensetzung nach Anspruch 7 oder der Emulsion nach Anspruch 10 in Kosmetikprodukten.

12. Kosmetikprodukt, umfassend den Polyester nach einem der Ansprüche 1 bis 3 oder die Zusammensetzung nach Anspruch 7 oder die Emulsion nach Anspruch 10 und einen Wirkstoff und/oder ein Pigment oder einen Farbstoff.

## Claims

1. Polyester capable of being obtained by a process comprising a step of reacting several reagents, the reagents comprising:
- at least one polyol,
- at least one dicarboxylic acid other than an acid dimer, and
- at least one acid trimer, in a mixture or not in a mixture with at least one acid dimer, it being understood that if an acid dimer is present, the mixture of acid trimer and acid dimer contains at least 50% by weight of acid trimer, the percentage by weight being given relative to the total weight of said mixture,
the acid trimer content being greater than or equal to 5% by weight, the percentage by weight being given relative to the total weight of the reagents reacted.

2. Polyester according to claim 1, **characterized in that** the polyol content is greater than or equal to 30% by weight, the percentage by weight being given relative to the total weight of the reagents reacted.

3. Polyester according to any one of the preceding claims, in which the dicarboxylic acid content, apart from the acid dimer, is greater than or equal to 35% by weight, the percentage by weight being given relative to the total weight of the reagents reacted.

4. Process for the preparation of a polyester, comprising a step of reacting several reagents, the reagents comprising a polyol, a dicarboxylic acid other than an acid dimer, and at least one acid trimer, in a mixture or not in a mixture with at least one acid dimer, it being understood that if an acid dimer is present, the mixture of acid trimer and acid dimer contains at least 50% by weight of acid trimer, the percentage by weight being given relative to the total weight of said mixture, the acid trimer content being greater than or equal to 5% by weight, the percentage by weight being given relative to the total weight of the reagents reacted.

5. Process for the preparation of a polyester, comprising a step of reacting several reagents, the reagents comprising a polyol, a dicarboxylic acid other than an acid dimer, and a mixture of acid trimer and acid dimer containing at least 50% by weight of acid trimer, the percentage by weight being given relative to the total weight of the mixture.

6. Process according to claim 4 or 5, carried out without a catalyst.

7. Composition comprising the polyester according to any one of claims 1 to 3, and a fat.

8. Use of the polyester according to any one of claims 1 to 3 or of the composition according to claim 7, as a thickening agent.

9. Use of the polyester according to any one of claims 1 to 3 or of the composition according to claim 7, as a shear-thinning agent.

10. Emulsion comprising the polyester according to any one of claims 1 to 3 or the composition according to claim 7, and water.

11. Use of the polyester according to any one of claims 1 to 3, or the composition according to claim 7, or the emulsion according to claim 10, in cosmetic products.

12. Cosmetic product comprising the polyester according to any one of claims 1 to 3, or the composition according to claim 7, or the emulsion according to claim 10, and an active ingredient and/or a pigment or a colorant.
